# EUROPEAN PATENT SPECIFICATION

(11) **EP 1 836 285 B1**
(45) Date of publication and mention of the grant of the patent: **20.11.2019**
(21) Application number: 05857106.8
(22) Date of filing: 16.12.2005
(51) Int. Cl.: C10L 1/06, C07C 2/56, C10G 29/20, C10G 50/00

(54) **INTEGRATED ALKYLATION PROCESS USING IONIC LIQUID CATALYSTS**
INTEGRIERTES ALKYLIERUNGSVERFAHREN UNTER VERWENDUNG VON IONISCHEN FLÜSSIGKEITEN ALS KATALYSATOREN
PROCEDE D'ALKYLATION INTEGRE METTANT EN OEUVRE DES CATALYSEURS LIQUIDES IONIQUES

(30) Priority: 21.12.2004 US 21167
(43) Date of publication of application: 26.09.2007
(73) Proprietor: Chevron U.S.A. Inc., San Ramon, CA 94583 (US)
(72) Inventor: TIMKEN, Hye Kyung C., Albany, California 94706 (US); ELOMARI, Saleh, Fairfield, California 94534 (US); TRUMBULL, Steve, San Leandro, California 94577 (US); CLEVERDON, Robert, Walnut Creek, California 94598 (US)
(74) Representative: Haseltine Lake Kempner LLP
(86) International application number: PCT/US2005/045707
(87) International publication number: WO 2006/073749

(56) References cited:
- GB-A- 558 487
- US-A- 5 654 251
- US-A- 5 702 589
- US-A- 5 705 729
- US-A- 5 750 455
- US-A- 6 096 939
- US-B1- 6 235 959

## Description

### FIELD OF THE INVENTION

The present invention relates to an integrated refining process for the production of high quality gasoline blending components from low value components.

### BACKGROUND OF THE INVENTION

Modern refineries employ many upgrading units such as fluidic catalytic cracking (FCC), hydrocracking (HCR), alkylation, and paraffin isomerization. As a result, these refineries produce a significant amount of isopentane. Historically, isopentane was a desirable blending component for gasoline having a high octane (92 RON), although it exhibited high volatility (20.4 Reid vapor pressure (RVP)). As environmental laws began to place more stringent restrictions on gasoline volatility, the use of isopentane in gasoline was limited because of its high volatility. As a consequence, the problem of finding uses for by-product isopentane became serious, especially during the hot summer season. Moreover, as more gasoline compositions contain ethanol instead of MTBE as their oxygenate component, more isopentane must be kept out of the gasoline pool in order to meet the gasoline volatility specification. So, the gasoline volatility issue becomes even more serious, further limiting the usefulness of isopentane as a gasoline blending component.

The process of the present invention solves this problem by converting undesirable isopentane to low-RVP gasoline blending components by alkylation of the isopentane with a refinery stream containing ethylene using an ionic liquid catalyst. Other olefins, such as propylene, butylenes, and pentenes can also be used to convert isopentane to make low RVP hydrocarbon product. By reducing the excess isopentane, the burden of storing isopentane and/or concerns for isopentane usage are eliminated.

In general, conversion of light paraffins and light olefins to more valuable cuts is very lucrative to the refining industries. This has been accomplished by alkylation of paraffins with olefins, and by polymerization of olefins. One of the most widely used processes in this field is the alkylation of isobutane with C₃-C₅ olefins to make gasoline cuts with high octane number using sulfuric and hydrofluoric acids. This process has been used by refining industries since the 1940's. The process was driven by the increasing demand for high quality and clean burning high octane gasoline.

Commercial paraffin alkylation processes in modern refineries use either sulfuric acid or hydrofluoric acid as catalyst. Both of these processes require extremely large amounts of acid to fill the reactor initially. The sulfuric acid plant also requires a huge amount of daily withdrawal of spent acid for off-site regeneration. Then the spent sulfuric acid is incinerated to recover SO₂/SO₃ and fresh acid is prepared. The necessity of having to handle a large volume of used acid is considered a disadvantage of the sulfuric acid based processes. On the other hand, an HF alkylation plant has on-site regeneration capability and daily make-up of HF is orders of magnitude less. However, the aerosol formation tendency of HF presents a potentially significant environmental risk and makes the HF alkylation process less safe than the H₂SO₄ alkylation process. Modern HF processes often require additional safety measures such as water spray and catalyst additive for aerosol reduction to minimize the potential hazards.

Although these catalysts have been successfully used to economically produce the best quality alkylates, the need for safer and environmentally-more friendly catalyst systems has become an issue to the industries involved. The ionic liquid catalyst of the present invention fulfills that need.

In addition, implementing the present invention relieves a refinery of the problem and waste associated with excess fuel gas production. It does this by using ethylene in, for example, offgas from a FCC unit as the source of olefins for the alkylation of isopentane. Typically FCC offgas contains ethylene up to 20 vol%. Other olefin streams containing ethylene or other olefins such as coker gas could also be used for this process. The overall gasoline volume is increased by this process of invention. The net amount of fuel gas from the FCC de-ethanizer is reduced, thus lowering the burden of fuel gas processing equipment. A further benefit of the present invention is that extracting ethylene will improve the purity of hydrogen in FCC offgas. The improved concentration of hydrogen in the offgas may allow the economical recovery of pure hydrogen with the use of a hydrogen recovery unit, such as a pressure-swing adsorption (PSA) unit or a selective hydrogen-permeable membrane unit. Considering tight environmental regulations associated with fuel gas production and shortage of hydrogen in modern refineries, the benefits of fuel gas reduction and hydrogen production are highly desirable.

The most economical, thus most desirable, olefin streams are FCC de-ethanizer overhead containing hydrogen, methane, ethane, and ethylene, or coker gas containing olefins. The present process converts the isopentane stream to a low RVP dimethyl pentane and trimethyl butane gasoline fraction with little octane loss. By employing the process of the invention, the overall gasoline volume produced at a refinery is increased. In addition, the net amount of fuel gas from the FCC de-ethanizer is reduced, thus lowering the burden on the fuel gas processing equipment.

Furthermore, the present invention includes a new paraffin alkylation process which can produce alkylate gasoline, the most desirable blending component in gasoline, in an environmentally sound manner far superior to the conventional alkylation process. In comparison with the conventional processes, the process according to the present invention offers the following significant advantages over conventional alkylation:
- Substantial reduction in capital expenditure as compared to sulfuric acid and hydrofluoric acid alkylation plants
- Substantial reduction in operating expenditure as compared to sulfuric acid alkylation plants
- Substantial reduction in catalyst inventory volume (potentially by 90%)
- A substantially reduced catalyst make-up rate (potentially by 98% compared to sulfuric acid plants)
- A higher gasoline yield
- Comparable or better product quality (Octane number, RVP, T50)
- Significant environment, health and safety advantages
- Expansion of alkylation feeds to include isopentane and ethylene.

It follows that employing a process according to the present invention a refiner can upgrade both isopentane and ethylene and at the same time react conventional alkylation feed components (e.g., butene, propylene, pentene, and isobutane) to produce high quality gasoline blending components. These additional capabilities are made possible in part with the high activity and selectivity of the ionic liquid catalyst used for these reactions. The present invention provides its greatest benefits when all these alkylation reactions are conducted with ionic liquid catalysts and none are conducted using sulfuric acid or hydrofluoric acid catalysts.

US 6235959 describes a process for alkylating isoparaffins by olefins using a catalyst being a composition resulting from a mixture of at least aluminum halide, at least one quaternary ammonium halide and/or at least one amine hydrohalide and at least one group IVB metal compound.

US 5750455 describes a process for the alkylation of isoparaffins by olefins with a catalytic composition resulting from mixing at least one aluminum halide, at least one quaternary ammonium halide and/or at least one amine halohydrate and at least one cuprous compound.

### SUMMARY OF THE INVENTION

The present invention provides an integrated refinery process for the production of high quality gasoline blending components having low volatility comprising:
(a) providing a first ethylene-containing refinery stream;
(b) separating a C₂₊ fraction from said first stream to produce a second refinery stream richer in ethylene than said first stream;
(c) providing an isopentane-containing refinery stream;
(d) contacting said isopentane-containing refinery stream with said second refinery stream in the presence of an ionic liquid catalyst in an alkylation zone under alkylation conditions, wherein the ionic liquid catalyst comprises a hydrocarbyl substituted pyridinium chloride or hydrocarbyl substituted imidazolium chloride, and wherein the ionic liquid catalyst further comprises an HCI co-catalyst; and
(e) recovering high quality gasoline blending components of low volatility from said alkylation zone.

In addition the present invention provides a method of improving the operating efficiency of a refinery by reducing fuel gas production and simultaneously producing high quality gasoline blending components of low volatility.

Also disclosed herein is a high quality gasoline blending composition having low volatility prepared by the process described.

### BRIEF DESCRIPTION OF THE DRAWINGS

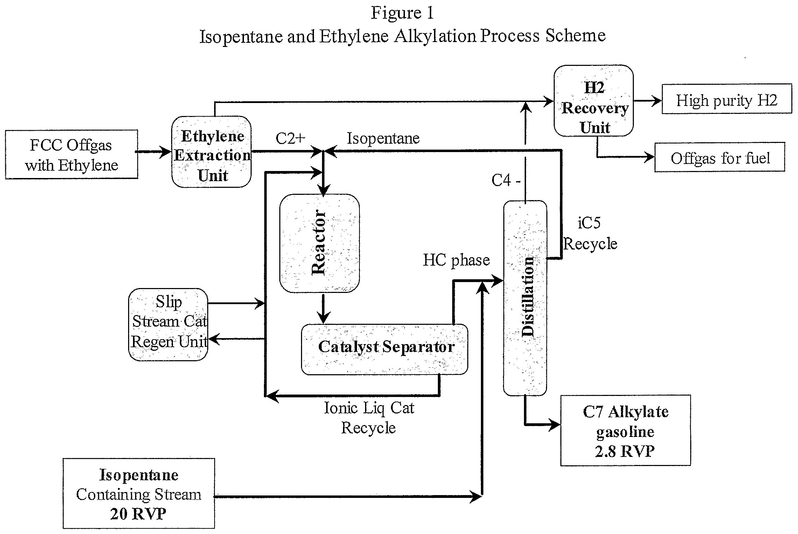

FIG. 1 is a diagram of for an integrated refinery process according to the present invention.

### DETAILED DESCRIPTION

### Feedstocks

One of the feedstocks to the process of the present invention is a refinery stream which contains olefins. Examples of such streams include FCC offgas, coker gas, olefin metathesis unit offgas, polyolefin gasoline unit offgas, methanol to olefin unit offgas and methyl-t-butyl ether unit offgas. The preferred olefin is ethylene. The preferred source of ethylene for conducting a process according to the present invention is offgas from an FCC unit, which may contain up to about 20 vol% of ethylene. This stream may also contain propylene, butylenes and pentenes. The FCC offgas is preferably passed through an ethylene extraction unit to produce a C₂₊ fraction, which is rich in ethylene, typically about 50 vol%, and a lighter fraction, which is rich in hydrogen. The C₂₊ fraction is fed to the alkylation reactor.

Another feedstock to the process of the present invention is a refinery stream which contains isoparaffins, preferably isopentane. Refinery streams which contain isopentane and which may be used in the process of the present invention include, but are not limited to extracted isopentane from an FCC unit, a hydrocracking unit, C₅ and C₆ streams from crude unit distillation, and extracted C₅ and C₆ streams from a reformer. Analysis of an extracted pentane sample from one refinery showed the feed stock to contain 86.4% iso-pentane, 8% n-pentane, 0.9% n-butane, 3.4% C₆s-C₉s and 0.2% olefins (C₄ and C₅ olefins). It also contained 88 ppm sulfur (mercaptans) and 0.4 ppm nitrogen. The feed stream exhibited very high RVP of 20, while the desirable current RVP target for gasoline is 7 to 8 range.

The isopentane-containing stream may also contain other isoparaffins such as isobutane. Isobutane may be obtained, for example, from hydrocracking units or may be purchased.

### Catalyst

The use of ionic liquids as a new media and solvents for chemical reactions and particularly catalytic processes has gained wide popularity in the past several years. There has been an overwhelming surge in this research arena where ionic liquids have been used as solvents in an array of reactions such as olefins dimerization, olefin oligomerization and polymerization, isomerizations, alkylations, hydrogenations, Diels-Alder cyclizations and many others. In short, ionic liquids have been used as solvents in a wide range of organic reactions and processes.

A large number of liquid or solid acid catalysts are known which are capable of effecting alkylation of isoparaffins such as isobutane or isopentane by olefins such as propylene, 1-butene, 2-butene and isobutylene. The catalysts which are most widely used in industrial practice are concentrated sulfuric acid and hydrofluoric acid alone or mixed with Lewis acids such as boron trifluoride.

Those processes suffer from major disadvantages: hydrofluoric acid by virtue of its toxicity and its high degree of volatility; and sulfuric acid by virtue of a substantial volumetric consumption of the catalyst requiring burdensome regeneration. These reasons have motivated the development of catalysts which are solid or which are supported on solids such as aluminosilicates or metal oxides such as zirconia treated with sulfuric acid. However, solid catalysts are generally found to present a low level of selectivity and a low degree of activity. The use of aluminum chloride has also been studied and proposed.

The process according to the present invention preferably employs a catalytic composition comprising at least one aluminum halide and at least one quaternary ammonium halide and/or at least one amine halohydrate. The aluminum halide which can be used in accordance with the invention is most preferably aluminum chloride.

The quaternary ammonium halides which can be used in accordance with the invention are those described in U.S. Patent No. 5,750,455, which also teaches a method for the preparation of the catalyst.

The ionic liquid catalysts which are most preferred for the process of the present invention are N-butylpyridinium chloroaluminate (C₅H₅NC₄H₉Al₂Cl₇). A metal halide may be employed as a co-catalyst to modify the catalyst activity and selectivity. Commonly used halides for such purposes include NaCl, LiCl, KCl, BeCl₂, CaCl₂, BaCl₂, SiCl₂, MgCl₂, PbCl₂, CuCl, ZrCl₄, AgCl, and PbCl₂ as published by Roebuck and Evering (Ind. Eng. Chem. Prod. Res. Develop., Vol. 9, 77, 1970). Preferred metal halides are CuCl, AgCl, PbCl₂, LiCl, and ZrCl₄.

HCI or any Broensted acid may be employed as an effective co-catalyst. The use of such co-catalysts and ionic liquid catalysts that are useful in practicing the present invention is disclosed in U.S. Published Patent Application Nos. 2003/0060359 and 2004/0077914. Other co-catalysts that may be used to enhance the catalytic activity of ionic liquid catalyst system include IVB metal compounds preferably metal halides such as TiCl₃, TiCl₄, TiBr₃, TiBr₄, ZrCl₄, ZrBr₄, HfCl₄, HfBr₄, as described by Hirschauer et al. in U.S. Patent No. 6,028,024.

It is especially important to note that H₂SO₄ and HF are not effective for the alkylation of isoparaffins with ethylene. So, the process of the present invention would not have been considered in the past.

### Reaction Conditions

Due to the low solubility of hydrocarbons in ionic liquids, olefins-isoparaffins alkylation, like most reactions in ionic liquids is generally biphasic and takes place at the interface in the liquid state. The catalytic alkylation reaction is generally carried out in a liquid hydrocarbon phase, in a batch system, a semi-batch system or a continuous system using one reaction stage as is usual for aliphatic alkylation. The isoparaffin and olefin can be introduced separately or as a mixture. The molar ratio between the isoparaffin and the olefin is in the range 1 to 100, for example, advantageously in the range 2 to 50, preferably in the range 2 to 20. In a semi-batch system the isoparaffin is introduced first then the olefin, or a mixture of isoparaffin and olefin. Catalyst volume in the reactor is in the range of 2 vol% to 70 vol%, preferably in the range of 5 vol% to 50 vol%. Vigorous stirring is desirable to ensure good contact between the reactants and the catalyst. The reaction temperature can be in the range -40°C to +150°C, preferably in the range -20°C to +100°C. The pressure can be in the range from atmospheric pressure to 8000 kPa, preferably sufficient to keep the reactants in the liquid phase. Residence time of reactants in the vessel is in the range a few seconds to hours, preferably 0.5 min to 60 min. The heat generated by the reaction can be eliminated using any of the means known to the skilled person. At the reactor outlet, the hydrocarbon phase is separated from the ionic phase by decanting, then the hydrocarbons are separated by distillation and the starting isoparaffin which has not been converted is recycled to the reactor.

Typical reaction conditions may include a catalyst volume in the reactor of 5 vol% to 50 vol%, a temperature of -10°C to 100°C, a pressure of 300 kPa to 2500 kPa, an isoparaffin to olefin molar ratio of 2 to 8 and a residence time of 1 min to 1 hour.

A catalyst system comprised of aluminum chloride and hydrogen chloride (hydrochloric acid) for catalyzing the alkylation of iso-paraffins and olefins in ionic liquids (chloroaluminate ionic liquids) is preferred. The HCl can be used as a co-catalyst to enhance the reaction rate. For example, the alkylation of isopentane with ethylene in a batch autoclave is complete in <10 minutes in the presence of HCl. In the absence of HCl, the reaction usually takes 1/2 hour to 1 hour time (50°C and autogenic pressure of ∼965 kPa and feed ratio of ∼4). The product selectivity was comparable to that of chloroaluminate ionic liquid without the presence of HCl.

### Process Configuration

A scheme for an integrated refinery alkylation process to implement an embodiment of the present invention is shown in Figure 1.

An ethylene-containing refinery stream is fed to an Ethylene Extraction Unit to separate a C₂₊ fraction rich in ethylene. The Ethylene Extraction Unit is typically comprised of membrane and/or distillation column separation equipment. A second refinery stream containing isopentane is fed to a Distillation Zone. Streams enriched in ethylene and isopentane are contacted in the presence of an ionic liquid catalyst in a Reactor under alkylation conditions. Then the catalyst and hydrocarbon phases are separated in a Catalyst Separator and the catalyst is recycled back to the Reactor. A portion of the recycling catalyst is sent to a Slip Stream Catalyst Regeneration unit. The hydrocarbon phase is sent to a Distillation Zone to recover unreacted isopentane for recycle, and the alkylate product is collected at the bottom. As needed, the alkylate product can be treated to remove any trace impurities.

The reject stream from the Ethylene Extraction Unit now has higher hydrogen purity. Further upgrading of the reject stream can be achieved by recovering pure hydrogen gas with use of a H₂ Recovery Unit if desirable. The H₂ Recovery Unit is typically comprised of a selective hydrogen-permeable membrane unit and/or pressure-swing adsorption (PSA) unit.

A process according to the present invention offers a refiner considerable flexibility with respect to being able to prepare gasoline blending components of varying composition by selecting both the source of olefins used for alkylation and the paraffin-containing feedstock. Alkylation reactions in accordance with the present invention may be conducted in one or more alkylation zone using the same or different ionic liquid catalysts. For example, the C₂₊ fraction described above may contain propylene, butylene and/or pentenes and the isopentane containing stream may also contain isobutane. Isobutane may be alkylated with ethylene to produce a high-octane C₆ gasoline blending component. A C₄ olefin containing stream may be isolated and used for the alkylation of isobutane, isopentane or their mixtures. Other variations and combinations will be apparent to refiners generally.

The following examples are illustrative of the present invention, but are not intended to limit the invention in any way beyond what is contained in the claims which follow.

### EXAMPLES

### Example 1: The Preparation of N-Butyl-Pyridinium Chloroaluminate Ionic Liquid

N-butyl-pyridinium chloroaluminate is a room temperature ionic liquid prepared by mixing neat N-butyl-pyridinium chloride (a solid) with neat solid aluminum trichloride in an inert atmosphere. The syntheses of butylpyridinium chloride and the corresponding N-butyl-pyridinium chloroaluminate are described below. In a 2-L Teflon-lined autoclave, 400 gm (5.05 mol.) anhydrous pyridine (99.9% pure purchased from Aldrich) were mixed with 650 gm (7 mol.) 1-chlorobutane (99.5% pure purchased from Aldrich). The neat mixture was sealed and let to stir at 145°C under autogenic pressure over night. Then, the autoclave was cooled down to room temperature, vented and the resultant mixture was transferred to a three liter round bottom flask. Chloroform was used to rinse the liner and dissolve the stubborn crusty product that adhered to the sides of the liner. Once all transferred, the mixture was concentrated at reduced pressure on a rotary evaporator (in a hot water bath) to remove excess chloride, un-reacted pyridine and the chloroform rinse. The obtained tan solid product was further purified by dissolving in hot acetone and precipitating the pure product through cooling and addition of diethyl ether. Filtering and drying under vacuum and heat on a rotary evaporator gave 750 gm (88% yields) of the desired product as an off-white shinny solid. 1H-NMR and 13C-NMR were ideal for the desired N-butyl-pyridinium chloride and no presence of impurities was observed by NMR analysis. N-butylpyridinium chloroaluminate was prepared by slowly mixing dried N-butylpyridinium chloride and anhydrous aluminum chloride (AlCl₃) according to the following procedure. The N-butylpyridinium chloride (prepared as described above) was dried under vacuum at 80°C for 48 hours to get rid of residual water (N-butylpyridinium chloride is hydroscopic and readily absorbs water from exposure to air). Five hundred grams (2.91 mol.) of the dried N-butylpyridinium chloride were transferred to a 2-Liter beaker in a nitrogen atmosphere in a glove box. Then, 777.4 gm (5.83 mol.) of anhydrous powdered AlCl₃ (99.99% from Aldrich) were added in small portions (while stirring) to control the temperature of the highly exothermic reaction. Once all the AlCl₃ was added, the resulting amber-looking liquid was left to gently stir overnight in the glove box. The liquid was then filtered to remove any un-dissolved AlCl₃. The resulting acidic N-butyl-pyridinium chloroaluminate was used as the catalyst for the alkylation of isopentane with ethylene.

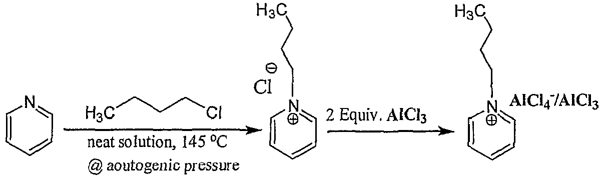

### Reference

### Example 2: Batch Alkylation Run Procedure

Isopentane and ethylene batch alkylation was typically run at 50°C with paraffin/olefin molar ratio of about 4. Under nitrogen atmosphere in a glove box, an autoclave vessel was charged with ionic liquid catalyst and anhydrous isopentane. The autoclave was then sealed and transferred to a hood and affixed to an overhead stirrer. Then, ethylene gas was introduced to the vessel. The autogenic pressure of the vessel usually rises to 2000 kPa to 24000 kPa depending on the amount of ethylene gas introduced into the autoclave. Once the reaction begins stirring (∼1200 rpm), the pressure quickly drops down to ∼900 kPa to 1100 kPa. The reaction is allowed to continue and stir until the pressure drops to 0 kPa to 70 kPa. Then, the stirring is stopped and the heating mantle is quickly removed. The autoclave is then cooled down to room temperature using a cooling coil. Then, a gas sample was drawn and the reactor is vented and weathered to relieve the system from any remaining gas. The resulting solution is a biphasic with the product and excess isopentane phase is on top while the dense ionic liquid-catalyst phase is on the bottom. The top phase is then decanted and saved for analysis. The bottom phase is either recycled for further use or neutralized with water. Chemical analysis of the products in excess isopentane is usually done by gas chromatography analysis.

### Reference

### Example 3: Batch Alkylation of Isopentane in Butylpyridinium Chloroaluminate without Applying Any Additional Pressure (Only the Autogenic Pressure of the System)

Ethylene (9.5 gm) was alkylated with isopentane (103 gm) in 20 gm butylpyridinium chloroaluminate ionic at 50°C and the autogenic pressure in a closed 300 cc autoclave fitted with an overhead stirrer and a cooling coil. The reaction was allowed to stir at ∼1200 rpm until no significant drop in pressure was noticeable. Table 1 below shows the reaction results.

### Example 4: Batch Alkylation of Isopentane in Butylpyridinium Chloroaluminate at Autogenic Pressure in the Presence of HCl as a Co-Catalyst

The reaction above was repeated in a fresh ionic liquid (19.6 gm) but this time HCl (0.35 gm) was added as a co-catalyst (promoter) with 102.7 gm isopentane and 9.7 gm ethylene. The reaction was run at 50°C and autogenic pressure and 1200 rpm stirring. The reaction was terminated when no further pressure drop was noticeable. With HCl the reaction was noticeably exothermic. Table 1 below shows the results of the reaction.

**Table 1**

| Batch Alkylation of Isopentane and Ethylene with ButylPyridinium Chloroaluminate Catalyst | | |
|---|---|---|
| | **Example 3** | **Example 4** |
| **Reaction** | **Without HCl** | **With HCl** |
| **iC5/C₂=** | 4 | 4 |
| **Temp. (°C)** | 50 | 50 |
| **Starting pressure, kPa** | 2050 | 2080 |
| **Ending Pressure, kPa** | 76 | 48 |
| **Reaction Time (min.)** | 44 | 5 |

| **Yields %** | | |
|---|---|---|
| **C₃₋** | 0 | 0 |
| **C₄** | 3.6 | 4.1 |
| **C₆** | 4.1 | 8.0 |
| **C₇** | 70.5 | 63.3 |
| **C₈** | 8.9 | 9.1 |
| **C₉** | 6.2 | 7.1 |
| **C₁₀** | 3.5 | 4.2 |
| **C₁₁₊** | 3.4 | 4.3 |

The results from isopentane/ethylene alkylation are excellent and most of the products are in the desired alkylates range where C₇s constitute the major fraction of the product mixture. Very little heavy products were produced.

Example 4 shows that addition of HCl as a co-catalyst enhances the activity of the ionic liquid catalyst and changes the product selectivity. When HCl was added as a co-catalyst, the reaction was done at much shorter time (completed in 5 minutes) and slight change in product selectivity was observed.

### Reference

### Example 5: Batch Alkylation of Isopentane and Ethylene with Other Chloroaluminate Ionic Liquid Catalyst

Other chloroaluminate ionic liquid catalysts with quaternary ammonium or amine halide salt can perform the same alkylation chemistry. Table 2 below compares the alkylation results of isopentane with ethylene in different chloroaluminate ionic liquid catalysts. Quaternary ammonium or amine salts used are 1-butyl-pyridinium (BPy), 4-methyl-1-butyl-pyridinium (MBPy), 1-butyl-4-methyl-imidaazolium (BMIM) and tributyl-methyl-ammonium (TBMA) chloroaluminates. The reactions were all conducted at 50°C and autogenic pressure at a feed paraffin/olefin molar ratio of 4, in 20 gm ionic liquid for 1 hour.

**Table 2**

| Batch Alkylation of Isopentane and Ethylene with Various Chloroaluminate Catalyst | | | | |
|---|---|---|---|---|
| **Salt used to make the chloroaluminate catalyst** | **MBPy** | **BPy** | **TBMA** | **BMIM** |
| **Starting Pressure, kPa** | 2040 | 2230 | 2140 | 1920 |
| **Ending Pressure, kPa** | 290 | 76 | 540 | 69 |
| | | | | |
| **Ethylene Conversion** | 65% | 95% | 55% | 95% |

| **Product Selectivity, wt%** | | | | |
|---|---|---|---|---|
| **C₃₋** | 2.6 | 0 | 3.0 | 0 |
| **C₄** | 3.3 | 3.6 | 2.4 | 3.6 |
| **C₆** | 3.8 | 4.3 | 2.7 | 4.2 |
| **C₇** | 65.8 | 65.6 | 69.1 | 68.8 |
| **C₈** | 9.9 | 9.8 | 9.2 | 9.7 |
| **C₉** | 7.3 | 6.5 | 7.3 | 6.4 |
| **C₁₀** | 5.5 | 4.7 | 4.3 | 4.3 |
| **C₁₁₊** | 1.6 | 3.4 | 1.9 | 3.0 |

The results above indicate that conversion of ethylene and product selectivity are affected by the catalyst selection. A chloroaluminate catalyst made with tributyl-methyl-ammonium is less active than the other three catalysts. Chloroaluminate catalysts made with hydrocarbyl substituted pyridinium chloride or a hydrocarbyl substituted imidazolium chloride shows high activity and good selectivity.

### Example 6: Continuous Alkylation of Isopentane with Ethylene

Evaluation of ethylene alkylation with isopentane was performed in a 100 cc continuously stirred tank reactor. 4:1 molar ratio of isopentane and ethylene mixture was fed to the reactor while vigorously stirring at 1600 rpm. An Ionic liquid catalyst was fed to the reactor via a second inlet port targeting to occupy 15 vol% in the reactor. A small amount of anhydrous HCl gas was added to the process (10:1 molar ratio of catalyst to HCl). The average residence time for the combined volume of feeds and catalyst was about 40 minutes. The outlet pressure was maintained at 2300 kPa using a backpressure regulator. The reactor temperature was maintained at 50°C. The reactor effluent was separated in a 3-phase separator into C₄₋ gas; alkylate hydrocarbon phase, and the ionic liquid catalyst. Operating conditions and yield information are summarized in Table 3.

**Table 3**

| Continuous Alkylation of Isopentane and Ethylene | |
|---|---|
| **Temperature, °C** | 50 |
| **Total Pressure, kPa** | 2300 |
| **Catalyst Volume Fraction** | 0.15 |
| **External I/O Ratio, molar** | 4.0 |
| **Olefin Space Velocity/Vol. of Cat (LHSV)** | 1.1 |
| **Catalyst to HCl Ratio, molar** | 10 |
| **Residence Time of Reactant, min** | 40 |
| **Conversion of Ethylene, wt%** | 95 |

| **Selectivity of Converted Products, wt%** | |
|---|---|
| **C₄₋** | 4.3 |
| **n C₅+ neo C₅** | 2.1 |
| **C₆** | 4.2 |
| **C₇** | 78.6 |
| **C₈** | 1.4 |
| **C₉** | 7.0 |
| **C₁₀₊** | 2.4 |
| **Total** | 100.0 |

| **C₇ Product Isomer Distribution, %** | |
|---|---|
| **Trimethylbutane/ total C₇** | 0.2 |
| **2,3-Dimethylpentane/ total C₇** | 49.0 |
| **2,4-Dimethylpentane/ total C₇** | 48.6 |
| **Other-Dimethylpentane/ total C₇** | 0.1 |
| **Methylhexane/ total C₇** | 2.1 |
| **n-heptane/ total C₇** | 0.0 |
| **Sum** | 100.0 |

This alkylation process is highly selective in that 78.6% of the converted product is C₇ isoparaffins. Detailed compositional analysis of the alkylate gasoline indicates the C₇ fraction is nearly entirely derived from 2,3- and 2,4-dimethylpentane. 2,3-dimethylpentane and tri-methylbutanes are desirable isomers for high-octane gasoline (91 and 112 RON, respectively).

The hydrocarbon product was distilled to separate n-pentane and higher boiling alkylate gasoline (30°C+) fraction and properties of the alkylate gasoline were measured or estimated. Research octane number was calculated based on GC composition and research octane number of pure compounds assuming volumetric linear blending. Blending octane numbers were measured at 7.5% and 15% blending level, then extrapolated to 100%. RVP and average density were estimated using the GC data assuming linear molar blending. T10, T50 and T90 were measured using ASTM D2887 simulated distillation.

**Table 4**

| Product Properties of Alkylate Gasoline from Isopentane and Ethylene Alkylation | |
|---|---|
| **Average density, g/cc** | 0.69 |
| **Average molecular weight, g/mole** | 104 |
| **Average RVP** | 2.5 |
| **Average RON** | 87 |
| **Blending RON** | 91 |
| **Blending MON** | 84 |
| | |

| **Simulated Distillation, D2887, °C** | |
|---|---|
| **T-10 wt%** | 76 |
| **T-50 wt%** | 88 |
| **T-90 wt%** | 119 |

The product property data shows that by employing the process of the present invention, high RVP isopentane (20 RVP) was converted to alkylate gasoline having a low RVP of 2.5. The high-octane (91 blending RON) and excellent boiling point distribution are other desirable features of the gasoline blending components prepared in accordance with the present method. To achieve the high-octane, it is preferable to maintain the 2,3-dimethylpentane selectivity above 40% relative to the total C₇ yield.

### Example 7: Continuous Alkylation of Isopentane with Propylene

Propylene alkylation with isopentane was performed via a similar procedure to that described in Example 6 except different process conditions were used. 8:1 molar ratio of isopentane and propylene mixture was fed to the reactor, at 10°C reactor temperature and 7 vol% of catalyst. A summary of operating conditions and yield information are presented in Table 5.

**Table 5**

| Continuous Alkylation of Isopentane and Propylene | |
|---|---|
| **Temperature, °C** | 10 |
| **Total Pressure, kPa** | 290 |
| **Catalyst Volume Fraction** | 0.07 |
| **External I/O Ratio, molar** | 8.0 |
| **Olefin Space Velocity/Vol. of Cat (LHSV)** | 4.4 |
| | |
| **Residence Time of Reactant, min** | 24 |
| | |
| **Conversion of Propylene, wt%** | 100 |
| | |

| **Selectivity of Converted Products, wt%** | |
|---|---|
| **C₄₋** | 3.6 |
| **C₆** | 2.3 |
| **C₇** | 1.4 |
| **C₈** | 74.2 |
| **C₉** | 2.9 |
| **C₁₀₊** | 15.6 |
| **Total** | 100.0 |
| | |

| **C₈ Product Isomer Distribution, wt%** | |
|---|---|
| **Trimethylpentane/ total C₈** | 36.5 |
| **Dimethylhexane/ total C₈** | 54.8 |
| **Methylheptane/ total C₈** | 8.7 |
| **n-octane/ total C₈** | 0.0 |
| **Sum** | 100.0 |

The hydrocarbon product was distilled to generate n-pentane and higher boiling alkylate gasoline (30°C+) fraction and properties of the alkylate gasoline were measured or estimated, and reported in Table 6.

**Table 6**

| Product Properties of Alkylate Gasoline from Isopentane and Propylene Alkylation | |
|---|---|
| **Average density, g/cc** | 0.71 |
| **Average molecular weight, g/mole** | 119 |
| **Average RVP** | 1.0 |
| **Average RON** | 82 |
| **Blending RON** | 79 |
| **Blending MON** | 78 |
| | |

| **Simulated Distillation, D2887, deg C** | |
|---|---|
| **T-10 wt%** | 107 |
| **T-50 wt%** | 111 |
| **T-90 wt%** | 169 |

The product property data shows that employing a process according to the present invention high RVP isopentane (20 RVP) was converted to alkylate gasoline having a low RVP of 1.0. The high-octane (82 RON), and excellent boiling point distribution are other desirable features of gasoline blending components prepared in accordance with the present invention.

### Example 8: Alkylation of Isobutane with 2-Butene

Evaluation of C₄ olefin alkylation with isobutane was performed in a 100 cc continuously stirred tank reactor. 8:1 molar ratio of isobutane and 2-butene mixture was fed to the reactor while vigorously stirring at 1600 RPM. An Ionic liquid catalyst was fed to the reactor via a second inlet port targeting to occupy 10-15 vol% in the reactor. A small amount of anhydrous HCI gas was added to the process. The average residence time (combined volume of feeds and catalyst) was about 8 minutes. The outlet pressure was maintained at 100 psig using a backpressure regulator. The reactor temperature was maintained at 0 °C using external cooling. The reactor effluent was separated in a 3-phase separator into C₄⁻ gas, alkylate hydrocarbon phase, and the ionic liquid catalyst. Detailed composition of alkylate gasoline was analyzed using gas chromatography. Research Octane number was calculated based on GC composition and Research Octane number of pure compounds assuming volumetric linear blending. The operating conditions and performance are summarized in Table 7.

| Table 1: Paraffin Alkylation with C4 olefins | | |
|---|---|---|
| Feed Olefin Source | cis-2-butene | trans-2-butene |
| Feed Paraffin Source | isobutane | isobutane |
| | | |
| Catalyst | BupyAl2Cl7 | CuCl/BupyAl2Cl7 |
| AlCl3 cat: HCl molar ratio | 60 | 40 |
| | | |
| Acid volume fraction | 0.1 | 0.15 |
| RPM of reactor stirring | 1600 | 1600 |
| Temp | 0 | 0 |
| Olefin space velocity, LHSV | 6.6 | 4.3 |
| External I/O ratio, molar | 8.0 | 8.0 |
| Residence time of reactant, min | 8.0 | 8.1 |
| | | |
| Olefin conversion, wt% | 100 | 100 |
| | | |

| C5+ Gasoline Composition | | |
|---|---|---|
| C5 | 1.1 | 1.5 |
| C6 | 2.4 | 1.8 |
| C7 | 2.7 | 2.3 |
| C8 | 82.9 | 79.8 |
| C9+ | 10.9 | 14.6 |
| Sum | 100.0 | 100.0 |
| | | |
| % tri-Me-pentane/ total C8 | **95.3** | **95.3** |
| % Di-Me-hexane/ total C8 | 4.5 | 4.5 |
| % Me-Heptane/ total C8 | 0.2 | 0.2 |
| % n-Octane/ total C8 | 0.0 | 0.0 |
| | | |
| **Research Octane** | **98.6** | **98.4** |

The results in Table 7 show that high octane alkylate can be obtained with n-butylpyridinium chloroaluminate ionic liquid catalyst. With 2-butene, over 95% of the C8 fraction is composed of trimethylpentanes having a RON of about 100.

### Example 9: Fuel Gas Reduction and H₂ Recovery Option

The process of the present invention can decrease the amount of excess fuel gas production in a refinery by converting ethylene in FCC offgas. This aspect of this invention is shown in this Example using a typical FCC offgas data from a refinery, as summarized in Table 8.

**Table 8**

| Fuel Gas Reduction and H₂ Recovery Option using Ethylene Alkylation | | | |
|---|---|---|---|
| | **Typical FCC Offgas As-Is** | **After C₂₊ Extraction** | **After C₂₊ Extraction + H₂ Recovery** |
| **Offgas Volume, MMSCFD*** | 26 | 21 | 12 |
| | 0 | | |
| **Reduction in Fuel Gas, %** | **(Base case)** | **19** | **55** |
| | | | |
| **H₂ Recovered, MMSCFD** | 0 | 0 | **9.2** |
| | | | |
| **Offgas Composition, vol%** | | | |
| **H₂S** | 10 ppm | 0 | 0 |
| **N₂** | 6.0 | 7.4 | 13.2 |
| **O₂** | 0.1 | 0 | 0 |
| **CO₂** | 0.4 | 0 | 0 |
| **CO** | 0.3 | 0 | 0 |
| **H₂** | **35.8** | **44.0** | **0** |
| **Methane** | 27.5 | 33.8 | 60.4 |
| **Ethane** | 10.6 | 13 | 23.2 |
| **Ethylene** | 15 | 0 | 0 |
| **Propane** | 1.2 | 1.5 | 2.7 |
| **Propylene** | 2.5 | 0 | 0 |
| **n-Butane** | 0.1 | 0.1 | 0.2 |
| **Isobutane** | 0.1 | 0 | 0 |
| **Butene** | 0.1 | 0 | 0 |
| **C₅₊** | 0.3 | 0.2 | 0.4 |
| **Sum** | 100 | 100.0 | 100.0 |

This refinery generates 26 million standard cubic feet (MMSCFD) of fuel gas from an FCC unit daily and the stream contains 15 vol% ethylene. Using a process according to the present invention results in converting the ethylene stream into high-octane gasoline blending component by alkylating the stream with isopentane or isobutane. The amount of fuel gas from the ethylene extraction unit is reduced to 21 MMSCFM, thus lowering the burden of fuel gas processing equipment. In this case, approximately a 19 percent reduction of fuel gas is feasible.

Extracting ethylene or the C₂₊ stream will improve the purity of hydrogen in FCC offgas as shown in Table 8, from 36% to 44%. Further upgrading of the reject stream can be achieved by recovering pure hydrogen gas with use of a hydrogen recovery unit such as a pressure-swing adsorption (PSA) unit or a membrane unit. By combining extraction of ethylene and hydrogen recovery, the amount of fuel gas is reduced substantially. In this case, up to a 55% reduction of fuel gas can be realized relative to the base case. In addition, 9 MMSCFD of hydrogen gas can be recovered. Considering the very stringent environmental regulations that are associated with fuel gas production and storage of hydrogen in refineries, the benefits of fuel gas reduction and hydrogen production that are made possible by using the present invention are significant and highly desirable.

There are numerous variations on the present invention which are possible in light of the teachings and supporting examples described herein. It is therefore understood that within the scope of the following claims, the invention may be practiced otherwise than as specifically described or exemplified herein.

## Claims

1. An integrated refinery process for the production of high quality gasoline blending components having low volatility comprising:
(a) providing a first ethylene-containing refinery stream;
(b) separating a C₂₊ fraction from said first stream to produce a second refinery stream richer in ethylene than said first stream;
(c) providing an isopentane-containing refinery stream;
(d) contacting said isopentane-containing refinery stream with said second refinery stream in the presence of an ionic liquid catalyst in an alkylation zone under alkylation conditions, wherein the ionic liquid catalyst comprises a hydrocarbyl substituted pyridinium chloride or hydrocarbyl substituted imidazolium chloride, and wherein the ionic liquid catalyst further comprises an HCI co-catalyst; and
(e) recovering high quality gasoline blending components of low volatility from said alkylation zone.

2. A process according to claim 1, wherein the ethylene-containing refinery stream comprises offgas from a FCC unit.

3. A process according to claim 1, wherein the ethylene-containing refinery stream comprises FCC de-ethanizer overhead.

4. A process according to claim 1, wherein the ionic liquid catalyst comprises an alkyl substituted pyridinium chloride or an alkyl substituted imidazolium chloride.

5. A process according to claim 4, wherein the ionic liquid catalyst is selected from the group consisting of 1-butyl-4-methyl-pyridinium chloroaluminate (BMP), 1-butyl-pyridinium chloroaluminate (BP) and 1-butyl-3-methylimidazolium chloroaluminate (BMIM).

6. A process according to claim 1, further comprising blending the high quality gasoline blending components into gasoline.

7. A process according to claim 1, wherein the isopentane-containing stream comprises isopentane extracted from an FCC unit, isopentane extracted from a hydrocracking unit, C₅ and C₆ streams derived from distillation of crude oil, or C₅ and C₆ streams extracted from a reformer.

8. A process according to claim 1, wherein the first ethylene-containing refinery stream comprises ethylene, propylene, butylenes and pentenes.

9. A process according to claim 1, wherein the first refinery stream contains hydrogen and further comprises separating a C₂₋ fraction from the first refinery stream which is richer in hydrogen than said first stream; and recovering hydrogen from the C₂₋ fraction.

10. A process according to claim 1, further comprising:
(f) providing a third refinery stream comprising at least one olefin selected from the group consisting of ethylene, propylene, butylenes, pentenes and mixtures thereof;
(g) providing a fourth refinery stream comprising at least one isoparaffin selected from the group consisting of isobutane, isopentane and mixtures thereof;
(h) contacting said third and fourth refinery streams with an ionic liquid catalyst in a second alkylation zone under alkylation conditions; and
(i) recovering gasoline blending components from the second alkylation zone.

11. A method of improving the operating efficiency of a refinery by reducing fuel gas production and simultaneously producing high quality gasoline blending components of low volatility comprising:
(a) providing a first refinery stream comprising hydrogen and C₂-C₅ olefins;
(b) separating a C₂₊ fraction from said first stream to produce a second refinery stream richer in olefins than said first stream and a third refinery stream richer in hydrogen than said first stream;
(c) providing an isopentane-containing refinery stream;
(d) contacting said isopentane-containing refinery stream with said second refinery stream in the presence of an ionic liquid catalyst in an alkylation zone under alkylation conditions, wherein the ionic liquid catalyst comprises a hydrocarbyl substituted pyridinium chloride or hydrocarbyl substituted imidazolium chloride, and wherein the ionic liquid catalyst further comprises an HCl co-catalyst;
(e) recovering high quality gasoline blending components of low volatility from said alkylation zone; and
(f) recovering hydrogen from said third refinery stream.

12. A method according to claim 11, wherein the ethylene-containing refinery stream comprises offgas from a FCC unit.

13. A method according to claim 11, wherein the ethylene-containing refinery stream comprises FCC de-ethanizer overhead.

14. A process according to claim 11, wherein the ionic liquid catalyst comprises an alkyl substituted pyridinium chloride or an alkyl substituted imidazolium chloride.

15. A process according to claim 14, wherein the ionic liquid catalyst is selected from the group consisting of 1-butyl-4-methyl-pyridinium chloroaluminate (BMP), 1-butyl-pyridinium chloroaluminate (BP) and 1-butyl-3-methyl-imidazolium chloroaluminate (BMIM).

16. A method according to claim 11, further comprising blending the high quality gasoline blending components into gasoline.

17. A method according to claim 11, wherein the isopentane-containing stream comprises isopentane extracted from an FCC unit, isopentane extracted from a hydrocracking unit, C₅ and C₆ streams derived from distillation of crude oil, or C₅ and C₆ streams extracted from a reformer.

18. A method according to claim 11, wherein the first ethylene-containing refinery stream comprises ethylene, propylene, butylenes and pentenes.

19. A method according to claim 11, further comprising:
(g) providing a third refinery stream comprising at least one olefin selected from the group consisting of ethylene, propylene, butylenes, pentenes and mixtures thereof;
(h) providing a fourth refinery stream comprising at least one isoparaffin selected from the group consisting of isobutane, isopentane and mixtures thereof;
(i) contacting said third and fourth refinery streams with an ionic liquid catalyst in a second alkylation zone under alkylation conditions; and
(j) recovering gasoline blending components from the second alkylation zone.

## Patentansprüche

1. Integrierter Raffinerieprozess für die Herstellung von hochwertigen Benzinmischkomponenten mit geringer Flüchtigkeit, umfassend:
(a) Bereitstellen eines ersten ethylenhaltigen Raffinieriestroms;
(b) Trennen einer C₂₊-Fraktion vom ersten Strom, um einen zweiten Raffineriestrom zu erzeugen, der reicher an Ethylen ist als der erste Strom;
(c) Bereitstellen eines isopentanhaltigen Raffineriestroms;
(d) Zusammenbringen des isopentanhaltigen Raffineriestroms mit dem zweiten Raffineriestrom in der Anwesenheit eines lonenflüssigkeitskatalysators in einer Alkylierungszone unter Alkylierungsbedingungen, worin der lonenflüssigkeitskatalysator ein hydrocarbylsubstituiertes Pyridiniumchlorid oder ein hydrocarbylsubstituiertes Imidazoliumchlorid umfasst, und worin der lonenflüssigkeitskatalysator ferner einen HCl-Ko-Katalysator umfasst; und
(e) Rückgewinnen der hochwertigen Benzinmischkomponenten mit geringer Flüchtigkeit aus der Alkylierungszone.

2. Verfahren gemäß Anspruch 1, worin der ethylenhaltige Raffineriestrom das Abgas einer FCC-Einheit umfasst.

3. Verfahren gemäß Anspruch 1, worin der ethylenhaltige Raffineriestrom einen FCC-Overhead-Entethanisierer umfasst.

4. Verfahren gemäß Anspruch 1, worin der lonenflüssigkeitskatalysator ein alkylsubstituiertes Pyridiniumchlorid oder ein alkylsubstituiertes Imidazoliumchlorid umfasst.

5. Verfahren gemäß Anspruch 4, worin der lonenflüssigkeitskatalysator ausgewählt ist aus der Gruppe 1-Butyl-4-methylpyridiniumchloroaluminat (BMP), 1-Butylpyridiniumchloroaluminate (BP) und 1-Butyl-3-methylimidazolium-chloroaluminate (BMIM).

6. Verfahren gemäß Anspruch 1, ferner umfassend das Vermischen der hochwertigen Benzinmischkomponenten mit Benzin.

7. Verfahren gemäß Anspruch 1, worin der isopentanhaltige Raffineriestrom Isopentan umfasst, das aus einer FCC-Einheit extrahiert wurde, Isopentan, das aus einer Hydrocrackingeinheit extrahiert wurde, C₅- und C₆-Ströme, abgeleitet aus der Destillation von Rohöl, oder aus einem Reformer extrahierte C₅- und C₆-Ströme.

8. Verfahren gemäß Anspruch 1, worin der erste ethylenhaltige Raffineriestrom Ethylen, Propylen, Butylene und Pentene umfasst.

9. Verfahren gemäß Anspruch 1, worin der erste Raffineriestrom Wasserstoff enthält und ferner das Abtrennen einer C₂₋-Fraktion von dem ersten Raffineriestrom umfasst, der reicher an Wasserstoff ist als der erste Strom; und die Rückgewinnung von Wasserstoff aus der C₂₋-Fraktion.

10. Verfahren gemäß Anspruch 1, ferner umfassend:
(f) Bereitstellen eines dritten Raffineriestroms, umfassend mindestens ein Olefin, ausgewählt aus der Gruppe Ethylen, Propylen, Butylene, Pentene und deren Mischungen;
(g) Bereitstellen eines vierten Raffineriestroms, umfassend mindestens ein Isoparaffin, ausgewählt aus der Gruppe Isobutan, Isopentan und deren Mischungen;
(h) Zusammenbringen des dritten und des vierten Raffineriestroms mit einem lonenflüssigkeitskatalysator in einer zweiten Alkylierungszone unter Alkylierungsbedingungen; und
(i) Rückgewinnen der Benzinmischkomponenten aus der zweiten Alkylierungszone.

11. Verfahren für die Verbesserung der Betriebseffizienz einer Raffinerie durch Reduzierung der Brenngaserzeugung und gleichzeitiges Herstellen von hochwertigen Benzinmischkomponenten mit geringer Flüchtigkeit, umfassend:
(a) Bereitstellen eines ersten Raffineriestroms, umfassend Wasserstoff und C₂- bis C₅-Olefine;
(b) Abtrennen einer C₂₊-Fraktion vom ersten Strom, um einen zweiten Raffineriestrom herzustellen, der reicher an Olefinen ist als der erste Strom und einen dritten Raffineriestrom, der reicher an Wasserstoff ist als der erste Strom;
(c) Bereitstellen eines isopentanhaltigen Raffineriestroms;
(d) Zusammenbringen des isopentanhaltigen Raffineriestroms mit dem zweiten Raffineriestrom in der Anwesenheit eines lonenflüssigkeitskatalysator in einer Alkylierungszone unter Alkylierungsbedingungen, worin der lonenflüssigkeitskatalysator ein hydrocarbylsubstituiertes Pyridiniumchlorid oder ein hydrocarbylsubstituiertes Imidazoliumchlorid umfasst, und worin der lonenflüssigkeitskatalysator ferner einen HCl-Ko-Katalysator umfasst;
(e) Rückgewinnen der hochwertigen Benzinmischkomponenten mit geringer Flüchtigkeit aus der Alkylierungszone;
(f) Rückgewinnen von Wasserstoff aus dem dritten Raffineriestrom.

12. Verfahren gemäß Anspruch 11, worin der ethylenhaltige Raffineriestrom Abgas aus einer FCC-Einheit umfasst.

13. Verfahren gemäß Anspruch 11, worin der ethylenhaltige Raffineriestrom ein FCC-Entethanisierungs-Overhead umfasst.

14. Verfahren gemäß Anspruch 11, worin der lonenflüssigkeitskatalysator ein alkylsubstituiertes Pyridiniumchlorid oder ein alkylsubstituiertes Imidazoliumchlorid umfasst.

15. Verfahren gemäß Anspruch 14, worin der lonenflüssigkeitskatalysator ausgewählt ist aus der Gruppe 1-Butyl-4-methylpyridiniumchloroaluminat (BMP), 1-Butylpyridiniumchloroaluminat (BP) und 1-Butyl-3-methylimidazolium-chloroaluminat (BMIM).

16. Verfahren gemäß Anspruch 11, ferner umfassend das Vermischen der hochwertigen Benzinmischkomponenten mit Benzin.

17. Verfahren gemäß Anspruch 11, worin der isopentanhaltige Strom Isopentan umfasst, das aus einer FCC-Einheit extrahiert wurde, Isopentan, das aus einer Hydrocrackingeinheit extrahiert wurde, C₅- und C₆-Ströme, abgeleitet aus der Destillation von Rohöl, oder aus einem Reformer extrahierte C₅- und C₆-Ströme.

18. Verfahren gemäß Anspruch 11, worin der erste ethylenhaltige Raffineriestrom Ethylen, Propylen, Butylene und Pentene umfasst.

19. Verfahren gemäß Anspruch 11, ferner umfassend:
(g) Bereitstellen eines dritten Raffineriestroms, umfassend mindestens ein Olefin, ausgewählt aus der Gruppe Ethylen, Propylen, Butylene, Pentene und deren Mischungen;
(h) Bereitstellen eines vierten Raffineriestroms, umfassend mindestens ein Isoparaffin, ausgewählt aus der Gruppe Isobutan, Isopentan und deren Mischungen;
(i) Zusammenbringen des dritten und des vierten Raffineriestroms mit einem lonenflüssigkeitskatalysator in einer zweiten Alkylierungszone unter Alkylierungsbedingungen; und
(j) Rückgewinnen von Benzinmischkomponenten aus der zweiten Alkylierungszone.

## Revendications

1. Un procédé de raffinage intégré pour la production de composantes de mélange d'essence de haute qualité ayant une faible volatilité, comprenant:
(a) fournir un premier courant de raffinerie contenant de l'éthylène;
(b) séparer une fraction C₂₊ dudit premier courant pour produire un second courant de raffinerie plus riche en éthylène que ledit premier courant;
(c) fournir un courant de raffinerie contenant de l'isopropane;
(d) mettre en contact ledit courant de raffinerie contenant de l'isopentane avec ledit deuxième courant de raffinerie en présence d'un catalyseur de liquide ionique dans une zone d'alkylation dans des conditions d'alkylation, dans lequel le catalyseur de liquide ionique comprend un chlorure de pyridinium substitué par un hydrocarbyle ou du chlorure d'imidazolium substitué par un hydrocarbyle, et dans lequel le catalyseur de liquide ionique comprend en outre un co-catalyseur HCl; et
(e) récupérer les composantes de mélange d'essence de haute qualité à faible volatilité de ladite zone d'alkylation.

2. Un procédé selon la revendication 1, dans lequel le courant de raffinerie contenant de l'éthylène comprend un gaz d'échappement provenant d'une unité FCC.

3. Un procédé selon la revendication 1, dans lequel le courant de raffinerie contenant de l'éthylène comprend un déséthaniseur FCC en tête.

4. Un procédé selon la revendication 1, dans lequel le catalyseur de liquide ionique comprend un chlorure de pyridinium substitué par un alkyle ou un chlorure d'imidazolium substitué par un alkyle.

5. Un procédé selon la revendication 4, dans lequel le catalyseur ionique liquide est choisi parmi le groupe constitué en 1-butyl-4-méthyl-pyridinium chloroaluminate (BMP), 1-butyl-pyridinium chloroaluminate (BP) und 1-butyl-3-méthyl-imidazolium chloroaluminate (BMIM).

6. Un procédé selon la revendication 1, comprenant en outre mélanger des composantes de mélange d'essence de haute qualité dans l'essence.

7. Un procédé selon la revendication 1, dans lequel le courant contenant de l'isopentane comprend de l'isopentane extrait d'une unité FCC, de l'isopentane extrait d'une unité d'hydrocraquage, des courants C₅ et C₆ dérivés de la distillation de pétrole brut ou des courants C₅ et C₆ extraits d'un reformeur.

8. Un procédé selon la revindication 1, dans lequel le premier courant de raffinerie contenant de l'éthylène comprend de l'éthylène, du propylène, des butylènes et des pentènes.

9. Un procédé selon la revendication 1, dans lequel le premier courant de raffinerie contient de l'hydrogène et comprend en outre la séparation d'une fraction C₂₋ du premier courant de raffinerie, qui est plus riche en hydrogène que ledit premier courant; et la récupération de l'hydrogène de la fraction C₂₋.

10. Un procédé selon la revindication 1, comprenant en outre:
(f) fournir un troisième courant de raffinerie comprenant au moins une oléfine choisie parmi le groupe constitué en l'éthylène, le propylène, les butylènes, les pentènes et leurs mélanges;
(g) fournir un quatrième courant de raffinerie comprenant au moins une isoparaffine choisie parmi le groupe constitué en l'isobutane, l'isopentane et leurs mélanges;
(h) mettre en contact lesdits troisième et quatrième courants de raffinerie avec un catalyseur de liquide ionique dans une deuxième zone d'alkylation dans des conditions d'alkylation; et
(i) récupérer les composantes de mélange d'essence de la deuxième zone d'alkylation.

11. Un procédé pour améliorer l'efficacité opérationnelle d'une raffinerie en réduisant la production de gaz combustible et en produisant simultanément des composantes de mélange d'essence de haute qualité et à faible volatilité, comprenant:
(a) fournir un premier courant de raffinerie comprenant de l'hydrogène et des oléfines de C₂ à C₅;
(b) séparer une fraction C₂₊ dudit premier courant pour produire un deuxième courant de raffinerie plus riche en oléfines que ledit premier courant et un troisième courant de raffinerie plus riche en hydrogène que ledit premier courant;
(c) fournir un courant de raffinerie contenant de l'isopentane;
(d) mettre en contact ledit courant de raffinerie contenant de l'isopentane avec ledit deuxième courant de raffinerie en présence d'un catalyseur de liquide ionique dans une zone d'alkylation dans des conditions d'alkylation, dans lequel le catalyseur de liquide ionique comprend un chlorure de pyridinium substitué par un hydrocarbyle ou du chlorure d'imidazolium substitué par un hydrocarbyle, et dans lequel le catalyseur de liquide ionique comprend en outre un co-catalyseur HCl; et;
(e) récupérer les composantes de mélange d'essence de haute qualité à faible volatilité de ladite zone d'alkylation; et
(f) récupérer l'hydrogène dudit troisième courant de raffinerie.

12. Un procédé selon la revendication 11, dans lequel le courant de raffinerie contenant de l'éthylène comprend un gaz d'échappement provenant d'une unité FCC.

13. Un procédé selon la revendication 11, dans lequel le courant de raffinerie contenant de l'éthylène comprend un déséthaniseur FCC en tête.

14. Un procédé selon la revendication 11, dans lequel le catalyseur de liquide ionique comprend un chlorure de pyridinium substitué par un alkyle ou un chlorure d'imidazolium substitué par un alkyle.

15. Un procédé selon la revendication 14, dans lequel le catalyseur ionique liquide est choisi parmi le groupe constitué en 1-butyl-4-méthyl-pyridinium chloroaluminate (BMP), 1-butyl-pyridinium chloroaluminate (BP) et 1-butyl-3-méthyl-imidazolium chloroaluminate (BMIM).

16. Un procédé selon la revendication 11, comprenant en outre mélanger les composantes de mélange d'essence de haute qualité dans de l'essence.

17. Un procédé selon la revendication 11, dans lequel le courant contenant de l'isopentane comprend de l'isopentane extrait d'une unité FCC, de l'isopentane extrait d'une unité d'hydrocraquage, des courants C₅ et C₆ dérivés de la distillation de pétrole brut ou des courants C₅ et C₆ extraits d'un reformeur.

18. Un procédé selon la revindication 11, dans lequel le premier courant de raffinerie contenant de l'éthylène comprend de l'éthylène, du propylène, des butylènes et des pentènes.

19. Un procédé selon la revendication 11, comprenant en outre:
(g) fournir un troisième courant de raffinerie comprenant au moins une oléfine choisie parmi le groupe constitué en l'éthylène, le propylène, les butylènes, les pentènes et leurs mélanges;
(h) fournir un quatrième courant de raffinerie comprenant au moins une isoparaffine choisie parmi le groupe constitué en l'isobutane, l'isopentane et leurs mélanges;
(i) mettre en contact lesdits troisième et quatrième courants de raffinerie avec un catalyseur de liquide ionique dans une deuxième zone d'alkylation dans des conditions d'alkylation; et
(j) récupérer les composantes de mélange d'essence de la deuxième zone d'alkylation.
